(19) Europäisches Patentamt
European Patent Office
Office européen des brevets

(11) **EP 1 172 350 A2**

(12) **EUROPÄISCHE PATENTANMELDUNG**

(43) Veröffentlichungstag:
**16.01.2002 Patentblatt 2002/03**

(51) Int Cl.[7]: **C07C 51/215**, C07C 51/21,
C07C 53/21, C07C 57/56,
C07C 67/39, C07C 69/63,
C07C 69/635, C07C 69/65

(21) Anmeldenummer: **01116185.8**

(22) Anmeldetag: **04.07.2001**

(84) Benannte Vertragsstaaten:
**AT BE CH CY DE DK ES FI FR GB GR IE IT LI LU
MC NL PT SE TR**
Benannte Erstreckungsstaaten:
**AL LT LV MK RO SI**

(30) Priorität: **10.07.2000 DE 10033255**

(71) Anmelder: **Clariant GmbH
65929 Frankfurt am Main (DE)**

(72) Erfinder:
- **Grottenmüller, Ralf, Dr.
84489 Burghausen (DE)**
- **Knaup, Wolfgang, Dr.
84508 Burgkirchen (DE)**
- **Probst, Anton
84567 Erlbach (DE)**
- **Dullinger, Klaus
84508 Burgkirchen (DE)**

(54) **Verfahren zur Herstellung von Perfluorcarbonsäuren**

(57) Verfahren zur Herstellung von Perfluorcarbonsäuren der allgemeinen Formel $R_F\text{-COOH}$, deren Salze sowie deren Ester aus Perfluoralkyliodiden der allgemeinen Formel $R_F'\text{-I}$, worin $R_F$ und $R_F'$ cyclische, verzweigte oder lineare, gesättigte oder ungesättigte Perfluoralkylreste bedeuten, indem die Perfluoralkyliodide in Gegenwart von Sauerstoff und in organischen Lösungsmitteln aktiviert werden.

**Beschreibung**

Die Erfindung betrifft ein Verfahren zur Herstellung von Perfluorcarbonsäuren der Formel $R_F$-COOH, deren Salze sowie deren Ester aus Perfluoralkyliodiden der Formel $R_F'$-I durch Oxidation mit Sauerstoff unter Einwirkung von Licht, Metallen, Radikalquellen oder anderen Aktivatoren für Perfluoralkyliodide in organischen Lösungsmitteln. $R_F$ und $R_F'$ können cyclische, verzweigte oder lineare, gesättigte oder ungesättigte Perfluoralkylreste sein.

Perfluorcarbonsäuren sind chemisch und thermisch äußerst stabile Verbindungen mit ausgeprägten oberflächenaktiven Eigenschaften. Es besteht ein großer Bedarf an Perfluorcarbonsäuren, teilweise in hoher Reinheit, für eine Vielzahl unterschiedlicher Anwendungen. Ihre hervorragenden Emulgatoreigenschaften werden z.B. bei der Emulsionspolymerisation fluorierter Olefine wie z.B. Tetrafluorethylen ausgenutzt. So beschreibt DE-A-3512633 die Verwendung von Alkali- und Ammoniumsalzen der Perfluoroctansäure als Emulgator bei der Herstellung scherstabiler PTFE-Copolymer Dispersionen.

Es sind bereits mehrere Verfahren zur Herstellung von Perfluorcarbonsäuren bekannt, die jedoch alle gravierende Nachteile aufweisen.

Beispielsweise können Perfluorcarbonsäuren durch Hydrolyse von Perfluorcarbonsäurehalogeniden erhalten werden, die aus den entsprechenden fluorfreien Carbonsäurehalogeniden durch elektrochemische Umsetzung in flüssigem Fluorwasserstoff gewonnen werden. Der Umgang mit flüssigem Fluorwasserstoff, die mit steigender Kettenlänge stark abnehmende Ausbeute und die Bildung kettenverzweigter Umlagerungsprodukte schränken die Anwendbarkeit dieses Verfahrens ein.

DE-A-3606174 beschreibt die Oxidation von Perfluoralkylethylen der Formel $F(CF_2)_n$-CH=CH$_2$ mit Permanganat zu Perfluorcarbonsäuren. Dieses Verfahren hat den Nachteil, dass größere Mengen an Manganoxyden als Abfall anfallen. Auch das in US-4138417 beschriebene Ozon als Oxidationsmittel für Perfluoralkylethylen ist im technischen Maßstab nicht praktikabel.

In DE-A-2756169 wird die Umsetzung von Perfluoralkyliodid $F(CF_2)_n$-I mit Zink bzw. mit aktiviertem Zink und Kohlendioxyd zu Perfluorcarbonsäuren beschrieben. Die niedrige Ausbeute, die Notwendigkeit bestimmter Lösemittel sowie der Verbrauch an Zink sind Nachteile dieses Verfahrens.

DE-A-3043249 beschreibt die Oxidation von Perfluoralkyliodid $F(CF_2)_n$-I mit rauchender Schwefelsäure bei Temperaturen von 100 - 180°C. Die schwierige Handhabung von rauchender Schwefelsäure und die hohen Anforderungen an die chemische Beständigkeit der Apparate bereiten bei der technischen Durchführung dieses Verfahrens erhebliche Schwierigkeiten.

Es wurde nun ein Verfahren gefunden, das es erlaubt Perfluorcarbonsäuren in hoher Reinheit aus gut zugänglichem Perfluoralkyliodid unter milden Reaktionsbedingungen und unter Vermeidung größerer Mengen an Abfallprodukten herzustellen. Überraschenderweise wurde gefunden, dass sich Perfluoralkyliodide in organischen Lösungsmitteln unter Einwirkung von Licht oder anderen aktivierenden Substanzen oder Bedingungen in Gegenwart von Sauerstoff, wobei bereits Luftsauerstoff ausreicht, bereits bei Raumtemperatur in hohen Ausbeuten zu Perfluorcarbonsäuren hoher Reinheit umsetzen lassen. Als Perfluoralkyliodid kommen cyclische, verzweigte oder lineare, gesättigte oder ungesättigte Verbindungen in Frage. Beispiele für solche Verbindungen sind $F(CF_2)_i$-I, $(CF_3)_2$CF-$(CF)_j$-I, $F(CF_2)_k$-CF$(CF_3)$-I, $F(CF_2)_l$-CF$(CF_3)$-CF$_2$-I, $F(CF_2$-CF$(CF_3))_m$-I und F-$(CF_2$-CF$_2)_n$(CF$_2$-CF$(CF_3))_p$-I, wobei i, j, k, l, m, n und p jeweils ganze Zahlen von 0 bis 20 sind. Bevorzugte organische Lösungsmittel sind insbesondere niedrige Alkohole wie Methanol und Ethanol. Neben Licht kommen als Aktivatoren für Perfluoralkyliodid auch höhere Temperaturen, Metallkatalyse oder Radikalquellen wie z.B. Radikalstarter in Frage.

Das Perfluoralkyliodid wird in einer Konzentration von vorzugsweise 1-50 Gew.-% im organischen Lösemittel gelöst. Prinzipiell sind verschiedenste organische Lösemittel möglich. Als besonders geeignet haben sich jedoch kurzkettige lineare, verzweigte oder cyclische Alkohole mit $\leq 10$ Kohlenstoffatomen wie beispielsweise Methanol, Ethanol, Propanol, Butanol oder Isopropanol erwiesen. Die Perfluoralkyliodid-Lösung wird mit Sauerstoff begast. Es ist bereits ausreichend, Luft durch die Lösung zu leiten, obwohl reiner Sauerstoff die Reaktionsgeschwindigkeit erhöht. Zur weiteren Beschleunigung der Reaktion kann auch ein Überdruck an Luft oder Sauerstoff angelegt werden. Die Reaktion kann bei Raumtemperatur durch Bestrahlen mit Licht gestartet werden. Es ist bekannt, dass die Dissoziation von Perfluoralkyliodiden in Perfluoralkyradikal und Iodradikal photochemisch ausgelöst werden kann. Normales Tageslicht ist hierfür bereits ausreichend. Deutlich effektiver ist selbstverständlich die Bestrahlung mit UV-Licht z.B. mit Hilfe von Hg-Lampen. Vorzugsweise sollte die Wellenlänge des eingestrahlten Lichts im Bereich der UV-Absorption von Perfluoralkyliodiden bei ca. 250-310 nm liegen. Anstelle von Licht können auch Radikalquellen wie Azoverbindungen, Peroxyde, Percarbonate oder andere bekannte Radikalstarter verwendet werden, die thermisch oder durch chemische Reaktion freie Radikale erzeugen. Die Reaktion muss dann evtl. bei höheren Temperaturen entsprechend der Zerfallstemperatur des verwendeten Radikalstarters durchgeführt werden. Licht als Reaktionsauslöser bietet gegenüber Radikalstartern den Vorteil, dass keine störenden Bruchstücke zurückbleiben, ist aber im technischen Maßstab nur mit größerem Aufwand umsetzbar. Als weitere Möglichkeit die Reaktion auszulösen sind neben Licht und Radikalstartern noch bestimmte Metalle in homogener oder heterogener Form möglich. Es ist bekannt, dass Perfluoralkyliodide durch Metalle aktiviert werden können.

Beispielsweise läuft die Reaktion in Ethanol unter Einfluss von Licht, Metallkatalyse oder einer Radikalquelle nach folgender Summenformel ab:

$$2\ F(CF_2)_n\text{-}CF_2\text{-}I + 5\ CH_3CH_2\text{-}OH + O_2 \rightarrow 2\ F(CF_2)_n\text{-}CO\text{-}OCH_2CH_3 + I_2 + 4\ HF + CH_3CH(OCH_2CH_3)_2 + H_2O$$

Wird als Lösungsmittel Alkohol verwendet, entsteht aus dem bei der Reaktion primär gebildeten Perfluorcarbonsäurefluorid direkt der entsprechende Perfluorcarbonsäureester und der Alkohol wird überwiegend zum Aldehyd oxidiert, der mit überschüssigem Alkohol zum Acetal kondensiert. Teilweise findet auch weitergehende Oxidation des Lösemittels z.B. zur Carbonsäure statt.

Zur Aufarbeitung kann, falls ein wassermischbares Lösungsmittel vorliegt, nach Abtrennen des Iods mit Wasser verdünnt werden, worauf sich eine Unterphase abscheidet, die aus Perfluorcarbonsäure bzw. deren Ester und evtl. noch vorhandenem, nicht umgesetzten Perfluoralkyliodid besteht. Die Unterphase wird abgetrennt und destillativ gereinigt. Wurde das Iod vorher chemisch durch Reduktion oder Oxidation oder durch physikalische Methoden entfernt, kann die Reaktionslösung direkt durch fraktionierte Destillation aufgearbeitet werden. Das bei der Reaktion gebildete HF bzw. Fluorid kann durch Zugabe von Calciumsalzen wie z.B. $CaCl_2$, $CaCO_3$ oder $Ca(OH)_2$ als schwerlösliches $CaF_2$ ausgefällt und abfiltriert werden.

Die in den Tabellen 1 bis 3 beschriebenen Beispiele wurden, wenn nicht anders angegeben, nach folgender allgemeinen Vorschrift durchgeführt.

Perfluoroctyliodid ($C_8F_{17}I$, ® Fluowet I 800 - CLARIANT) wird im angegebenen Gewichtsverhältnis im Lösemittel gelöst. Die Versuche bei denen mit einer UV-Lampe belichtet wurde, wurden in einer wassergekühlten 250 ml Belichtungsapparatur aus Quarzglas durchgeführt. Die Versuche mit anderen Initiatoren als Licht wurden in einer abgedunkelten Apparatur durchgeführt. Über eine Kapillare wird die Lösung mit Luft begast. Es wird die angegebene Zeit bei der angegebenen Temperatur gerührt. Die Analytik erfolgt aus der Reaktionslösung ohne weitere Aufarbeitung durch $^{19}$F-NMR.

Tabelle 1

| Nr. | | $R_FI$ | LM : $R_FI$ g : g | Initiator | Zeit in h | Temp. in °C | Ergebnis | | | Sonstiges |
|---|---|---|---|---|---|---|---|---|---|---|
| | | | | | | | $R_FI$ | $R_FH$ | $R'_FCOR$ | |
| 1 | Ethanol | $C_8F_{17}I$ | 150 : 15 | Tageslicht | 76 | 25 | 87 % | < 1 % | 13 % | -- |
| 2 | Ethanol | $C_8F_{17}I$ | 100 : 10 | Tageslicht | 68 | 25 | > 99 % | < 1 % | < 1 % | unter $N_2$ Atmosphäre |
| 3 | -- | $C_8F_{17}I$ | 0 : 100 | Tageslicht | 50 | 25 | > 99 % | < 1 % | < 1 % | -- |
| 4 | Ethanol | $C_8F_{17}I$ | 150 : 15 | -- | 70 | 25 | > 99 % | < 1 % | < 1 % | -- |
| 5 | Ethanol | $C_8F_{17}I$ | 150 : 50 | UV – Lampe | 8 | 25 | 67 % | < 1 % | 33 % | -- |
| 6 | Methanol | $C_8F_{17}I$ | 200 : 30 | UV – Lampe | 5 | 40 | 84 % | 9 % | < 1 % | unter $N_2$ Atmosphäre |
| 7 | Methanol | $C_6F_{13}I$ | 150 : 50 | UV – Lampe | 8 | 25 | 61 % | < 1 % | < 39 % | -- |
| 8 | Methanol | $C_{10}F_{21}I$ | 150 : 50 | UV – Lampe | 8 | 25 | 64 % | < 1 % | < 36 % | -- |
| 9 | Methanol | $R_FI$-6* | 150 : 50 | UV – Lampe | 8 | 25 | 60 % | < 1 % | < 40 % | -- |
| 10 | Methanol | $R_FI$-8* | 150 : 50 | UV – Lampe | 8 | 25 | 68 % | < 1 % | < 32 % | -- |
| 11 | Methanol | i-$C_7F_{15}$** | 150 : 50 | UV – Lampe | 8 | 25 | 71 % | < 1 % | < 29 % | -- |

*$R_FI$-6: 44 % $C_6F_{13}I$, 35 % $C_8F_{17}I$, 15 % $C_{10}F_{21}I$, 6 % $\geq C_{12}F_{25}I$

*$R_FI$-8: 14 % $C_6F_{13}I$, 53 % $C_8F_{17}I$, 21 % $C_{10}F_{21}I$, 12 % $\geq C_{12}F_{25}I$

**i-$C_7F_{15}$: $(CF_3)_2CF(CF_2)_4$-I

Tabelle 1 zeigt, dass Perfluoroctyliodid unter Einfluss von Tageslicht bei Raumtemperatur (25°C) und bei Begasen mit Luft innerhalb von 76 Stunden 9 % Perfluoroctansäureethylester bildet (Nr.1). Wird sauerstofffrei unter $N_2$-Atmosphäre gearbeitet (Nr.2), ohne Lösungsmittel (Nr.3) oder ohne Einwirkung von Licht oder sonstiger Initiatoren (Nr.4) tritt keine Reaktion ein. Wenn anstelle von Tageslicht UV-Licht verwendet wird (Nr.5) verläuft die Reaktion deutlich schneller und in höherer Ausbeute. UV-Licht und $N_2$-Atmosphäre (Nr.6) führen zur Bildung von 1 H-Perfluoroctan. Die Reaktion verläuft bei Perfluoralkyliodiden verschiedener Kettenlängen bzw. mit Kettenschnitten (Nr.5, 7, 8, 9 und 10) und bei verzweigten Perfluoralkyliodiden (Nr.11) mit vergleichbarer Geschwindigkeit und Ausbeute.

Tabelle 2

| Nr. | | $R_F I$ | LM : $R_F I$ g : g | Initiator | Zeit in h | Temp. in °C | Ergebnis | | | Sonstiges |
|---|---|---|---|---|---|---|---|---|---|---|
| | | | | | | | $R_F I$ | $R_F H$ | $R'_F COR$ | |
| 1 | Ethanol | $C_8F_{17}I$ | 150 : 15 | UV – Lampe | 12 | 25 | 63 % | < 1 % | 37 % | -- |
| 2 | Isopropanol | $C_8F_{17}I$ | 150 : 15 | UV – Lampe | 12 | 25 | 65 % | < 1 % | 35 % | -- |
| 3 | Methanol | $C_8F_{17}I$ | 150 : 15 | UV – Lampe | 10 | 25 | 52 % | < 1% | 48 % | -- |
| 4 | Cyclohexan | $C_8F_{17}I$ | 150 : 15 | UV – Lampe | 10 | 25 | 93 % | < 1 % | 7 % | -- |
| 5 | Acetonitril | $C_8F_{17}I$ | 150 : 15 | UV – Lampe | 12 | 25 | 95 % | < 1 % | 5 % | -- |
| 6 | Ethylacetat | $C_8F_{17}I$ | 150 : 15 | UV – Lampe | 12 | 25 | 94 % | < 1 % | 6 % | -- |
| 7 | Glycerin | $C_8F_{17}I$ | 150 : 15 | UV – Lampe | 8 | 25 | 92 % | < 1 % | 8 % | -- |
| 8 | Cyclohexanol | $C_8F_{17}I$ | 150 : 15 | UV – Lampe | 8 | 25 | 79 % | < 1 % | 21 % | -- |
| 9 | tert.-Butanol | $C_8F_{17}I$ | 150 : 15 | UV – Lampe | 12 | 25 | 59 % | < 1 % | 41 % | -- |

Tabelle 2 zeigt den Einfluss verschiedener Lösemittel. Alkohole wie Methanol (Nr.3), Ethanol (Nr.1), Isopropanol (Nr.2), tert.-Butanol (Nr.9) und Cyclohexanol (Nr.8) ergeben in kurzer Zeit hohe Ausbeuten der gewünschten Perfluoroctylcarbonyl-Verbindungen. In Lösemittel die keine OH-Funktionen tragen wie Cyclohexan (Nr.4), Acetonitril (Nr.5) und Ethylacetat (Nr.6) sind die Umsätze unter vergleichbaren Bedingungen niedriger.

## Tabelle 3

| Nr. | | $R_FI$ | LM : $R_FI$ g : g | Initiator | Zeit in h | Temp. in °C | Ergebnis | | | Sonstiges |
|---|---|---|---|---|---|---|---|---|---|---|
| | | | | | | | $R_FI$ | $R_FH$ | $R'_FCOR$ | |
| 1 | Methanol | $C_8F_{17}I$ | 150 : 15 | -- | 24 | 25 | 98 % | < 1 % | < 1 % | Lichtausschluss |
| 2 | Methanol | $C_8F_{17}I$ | 150 : 15 | Tageslicht | 24 | 25 | 87 % | < 1 % | 13 % | -- |
| 3 | Methanol | $C_8F_{17}I$ | 150 : 20 | UV – Lampe | 12 | 25 | 52 % | < 1 % | 48 % | -- |
| 4 | Methanol | $C_8F_{17}I$ | 120 : 50 | VA 044*[1] (3 g) | 13 | 55 | 72 % | < 1 % | 28 % | Lichtausschluss |
| 5 | Methanol | $C_8F_{17}I$ | 200 : 60 | Perkadox 16S*[2] (3 g) | 8 | 45 | 51 % | 2 % | 47 % | Lichtausschluss |
| 6 | Methanol | $C_8F_{17}I$ | 200 : 60 | Perkadox 16S*[2] (5 g) | 9 | 45 | 33 % | 3 % | 64 % | Lichtausschluss |
| 7*[6] | Methanol | $C_8F_{17}I$ | 45 : 15 | Temp. / DTBP*[3] | 12 | 150 | < 1 % | 68 % | 32 % | Lichtausschluss |
| 8*[6] | Methanol | $C_8F_{17}I$ | 45 : 15 | Temp. | 12 | 100 | 40 % | 43 % | 17 % | Lichtausschluss |
| 9*[6] | Methanol | $C_8F_{17}I$ | 45 : 15 | Temp. | 12 | 150 | < 1 % | 90 % | 10 % | Lichtausschluss |
| 10*[6] | Methanol | $C_8F_{17}I$ | 45 : 15 | Temp. | 12 | 180 | < 1 % | > 99% | < 1 % | Lichtausschluss |
| 11 | Methanol | $C_8F_{17}I$ | 150 : 50 | Zink-Pulver | 5 | 25 | 55 % | 36 % | 9 % | Lichtausschluss |
| 12 | Methanol | $C_8F_{17}I$ | 100 : 15 | Fe-Pulver | 2 | 50 | 94 % | 1 % | 5 % | Lichtausschluss |
| 13 | Methanol | $C_8F_{17}I$ | 100 : 15 | Pd*[4] | 5 | 50 | 96 % | < 1 % | 4 % | Lichtausschluss |
| 14 | Methanol | $C_8F_{17}I$ | 250 : 50 | Cu-Pulver | 6 | 50 | 38 % | < 1 % | 62 % | Lichtausschluss |
| 15*[6] | Methanol | $C_8F_{17}I$ | 45 : 15 | Temp. / Cu-Pulver | 12 | 180 | < 1 % | 39 % | 71 % | Lichtausschluss |
| 16 | Methanol | $C_8F_{17}I$ | 70 : 15 | Cu- / Fe-Pulver | 5 | 50 | 66 % | < 1 % | 34 % | Lichtausschluss |
| 17*[6] | Methanol | $C_8F_{17}I$ | 60 : 15 | Pd*[4] | 12 | 120 | 3 % | 77 % | 20 % | Lichtausschluss |
| 18*[6] | Methanol | $C_8F_{17}I$ | 60 : 15 | Ru*[5] | 12 | 120 | 7 % | 74 % | 19 % | Lichtausschluss |

$*^1$ kationischer Azo-Radikalstarter

$*^2$ Peroxy-dicarbonat Radikalstarter

$*^3$ Di-tert.butyl-peroxyd

$*^4$ Typ E 106 R/W, Fa. Degussa

$*^5$ Typ H 105 RA, Fa. Degussa

$*^6$ Die Versuche bei Temperaturen größer 60 °C wurden in einem 250 ml

Stahlautoklaven mit Luftatmosphäre durchgeführt.

Tabelle 3 zeigt den Einfluss von unterschiedlichen Perfluoralkyliodid aktivierenden Substanzen oder Bedingungen.

Eine höhere Lichtintensität führt unter sonst vergleichbaren Bedingungen zu einer schnelleren Reaktion (Nr. 1, 2 und 3). Anstelle von Licht können auch üblicherweise für radikalische Reaktionen verwendete Radikalstarter bzw. Initiatoren eingesetzt werden (Nr.4, 5, 6 und 7). Der Umsatz an RFI hängt dabei von der Menge des eingesetzten Initiators ab (Nr.5 und 6). Bei Radikalstartern, die erst bei höheren Temperaturen freie Radikale erzeugen, wie z.B. Di-tert.-Butylperoxyd bei 150°C, entstehen größere Mengen an $R_FH$ (Nr.7). Temperaturen über 70°C ohne sonstige katalytisch wirkende Bedingungen liefern ebenfalls Perfluoroctylcarbonyl-Verbindungen, jedoch ist besonders ab 100°C eine bevorzugte Bildung von $R_FH$ zu beobachten (Nr.8, 9 und 10). Metalle wie z.B. Kupfer sind bereits bei Raumtemperatur katalytisch aktiv (Nr.14). Unedle Metalle wie Zink und Eisen ergeben niedrige Umsätze (Nr.12) oder bevorzugt $R_FH$ (Nr.11). Metalle wie Palladium (Nr.17) und Ruthenium (Nr.18) liefern bei höheren Temperaturen Produktgemische von Perfluoroctylcarbonyl-Verbindungen und $R_FH$.

Die folgenden zwei Beispiele beschreiben Möglichkeiten eine vollständige Umsetzung von Perfluoroctyliodid zu Perfluoroctansäuremethylester zu erzielen.

Beispiel 1: Katalyse mit UV-Licht.

In eine 500 Belichtungsapparatur aus Quarzglas mit Wasserkühlung und Hg-Lampe werden bei 25-30°C 75 g Perfluoroctyliodid und 300 ml Methanol gegeben. Durch eine Kapillare wird während der gesamten Reaktionszeit Luft eingegast. Nachdem sich die Lösung nach 4 h Rühren bei 25-30°C durch freiwerdendes Iod braun gefärbt hat, wird die Reaktion unterbrochen und die Lösung in ein 1 l Becherglas gegossen, wo sie mit 40 g Eisenpulver solange ausgerührt wird (ca. 15 min), bis die Farbe des Iods verschwunden ist. Nach Abfiltrieren des Niederschlags und des überschüssigen Eisenpulvers wird die jetzt klare Lösung in die Belichtungsapparatur zurückgegeben und die photochemische Umsetzung wird fortgesetzt. Der Reinigungsschritt muss im Verlauf der Reaktion nach jeweils 5 h noch 3 mal durchgeführt werden. Nach vollständigem Umsatz (Kontrolle durch $^{19}$F-NMR) wird die Lösung 5 x mit Wasser gewaschen und die fluorhaltige Unterphase wird fraktionierend destilliert.
Ausbeute: 53,9 g (92 %) Perfluoroctansäuremethylester als farblose Flüssigkeit, Siedepunkt 158 - 161°C.

Beispiel 2: Metall-Katalyse mit Kupfer.

Im einem beheizbaren 500 ml Dreihalskolben mit Rührwerk werden 75 g Perfluoroctyliodid und 300 ml Ethanol zusammen mit 20 g Kupfer-Pulver bei 50°C 12 h gerührt. Danach wird in Abständen von 6 h 8 mal jeweils 1,5 g frisches Kupfer-Pulver zugegeben. Nach vollständigem Umsatz (Kontrolle durch $^{19}$F-NMR) wird Abfiltriert und das Filtrat mehrmals mit Wasser gewaschen. Der Rückstand wird mit Schwefelsäure gründlich extrahiert. Die Schwefelsäurelösung wird eingeengt und die enthaltene Perfluoroctansäure durch Zugabe eines Überschusses Ethanol in den Ethylester überführt. Die vereinigten Fluorphasen werden fraktionierend destilliert.
Ausbeute: 52,7 g (87 %) Perfluoroctansäureethylester als farblose Flüssigkeit, Siedepunkt 180 - 184°C.

**Patentansprüche**

1.    Verfahren zur Herstellung von Perfluorcarbonsäuren der allgemeinen Formel $R_F$-COOH, deren Salze sowie deren Ester aus Perfluoralkyliodiden der allgemeinen Formel $R_F$'-I, worin $R_F$ und $R_F$' cyclische, verzweigte oder lineare, gesättigte oder ungesättigte Perfluoralkylreste bedeuten, indem die Perfluoralkyliodide in Gegenwart von Sauerstoff und in organischen Lösungsmitteln aktiviert werden.

2. Verfahren nach Anspruch 1, **dadurch gekennzeichnet, dass** die Aktivierung der Perfluoralkyliodide durch Licht, Radikalquellen, Temperatur oder Metalle in heterogener oder homogener Form bewirkt wird.

3. Verfahren nach Anspruch 1 und/oder 2, **dadurch gekennzeichnet, dass** Perfluoralkyliodide der Formeln $F(CF_2)_i$-I, $(CF_3)_2CF$-$(CF)_j$-I, $F(CF_2)_k$-$CF(CF_3)$-I, $F(CF_2)_l$-$CF(CF_3)$-$CF_2$-I, $F(CF_2$-$CF(CF_3))_m$-I und $F(CF_2$-$CF_2)_n(CF_2$-$CF(CF_3))_p$-I worin i, j, k, l, m, n und p jeweils unabhängig voneinander ganze Zahlen von 0 bis 20 bedeuten, verwendet werden.

4. Verfahren nach Anspruch 3, **gekennzeichnet durch** die Verwendung von Perfluoralkyliodiden der Formel $F(CF_2)_i$-I, wobei i eine ganze Zahl von 2 bis 16 ist, und bevorzugt i = 8 bedeutet.

5. Verfahren nach einem oder mehreren der Ansprüche 1 bis 4, **dadurch gekennzeichnet, dass** als organische Lösemittel Alkohole verwendet werden, bevorzugt niedrige verzweigte, cyclische oder lineare Alkohole mit bis zu 10 Kohlenstoffatomen.

6. Verfahren nach Anspruch 5, **dadurch gekennzeichnet, dass** bei der Herstellung der Perfluorcarbonsäuren in alkoholischen Lösungsmittel direkt die entsprechenden Perfluorcarbonsäureester entstehen.